(19) Europäisches Patentamt
European Patent Office
Office européen des brevets

(11) Veröffentlichungsnummer: **0 009 555**
**A1**

# (12) EUROPÄISCHE PATENTANMELDUNG

(21) Anmeldenummer: **79102572.9**

(22) Anmeldetag: **20.07.79**

(51) Int. Cl.³: **A 01 N 47/18**
A 01 N 25/32, C 07 C 103/34
C 07 C 121/43, C 07 D 205/04
C 07 D 207/06, C 07 D 209/02
C 07 D 209/52, C 07 D 211/16
C 07 D 265/04, C 07 D 295/18

(30) Priorität: **27.07.78 DE 2832974**

(43) Veröffentlichungstag der Anmeldung:
**16.04.80 Patentblatt 80/8**

(84) Benannte Vertragsstaaten:
**AT BE CH DE FR GB IT LU NL SE**

(71) Anmelder: **BASF Aktiengesellschaft**
**Carl-Bosch-Strasse 38**
**D-6700 Ludwigshafen(DE)**

(72) Erfinder: **Hansen, Hanspeter, Dr. Chem.**
**Thorwaldsenstrasse 5**
**D-6700 Ludwigshafen(DE)**

(72) Erfinder: **Eicken, Karl, Dr. Chem.**
**Waldstrasse 63**
**D-6706 Wachenheim(DE)**

(72) Erfinder: **Wuerzer, Bruno, Dr. Dipl.**
**Wilhelm-Busch-Strasse 55**
**D-6703 Limburgerhof(DE)**

## (54) Herbizide Mittel auf der Basis eines Thiolcarbamats, die Halogenacylamide als Antagonisten enthalten, und ihre Verwendung in einem Verfahren zur selektiven Bekämpfung von unerwünschtem Pflanzenwuchs.

(57) Die vorliegende Erfindung betrifft herbizide Mittel, gekennzeichnet durch einen Gehalt an Äthyl-N-äthyl-N-bicyclo- [2.2.1]hept-2-yl-thiolcarbamat der Formel

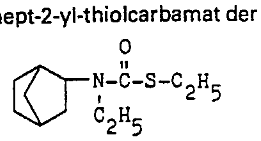

als herbizidem Wirkstoff und mindestens einem Halogenacylamid der Formel I

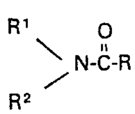
I

in der

R einen unverzweigten oder verzweigten Halogenalkylrest mit bis zu 3 Kohlenstoffatomen und

$R^1$ und $R^2$ gleich oder verschieden sind und einen unverzweigten oder verzweigten Alkylrest mit bis zu 6 Kohlenstoffatomen, der durch eine Alkoxygruppe mit bis zu 4 Kohlenstoffatomen oder eine Cyanogruppe substituiert sein kann, einen Cycloalkylrest mit 3 bis 6 Kohlenstoffatomen oder einen unverzweigten oder verzweigten Alkenyl- oder Alkinylrest mit bis zu 4 Kohlenstoffatomen bedeuten oder

$R^1$ und $R^2$ zusammen mit dem Stickstoffatom, dessen Substituenten sie sind, einen 4- bis 9-gliedrigen, gesättigten mono- oder bicyclischen Ring, der durch unverzweigte oder verzweigte Alkylreste mit bis zu 4 Kohlenstoffatomen einfach oder mehrfach substituiert sein kann, oder einen Tetrahydro-1,3-oxazinring der Formel

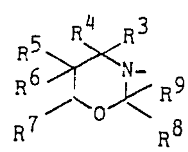

bilden,

in der

$R^3$, $R^4$, $R^5$, $R^6$ und $R^7$ gleich oder verschieden sind und Wasserstoff oder einen unverzweigten oder verzweigten Alkylrest mit bis zu 3 Kohlenstoffatomen,

$R^8$ Wasserstoff oder einen unverzweigten oder verzweigten

./...

EP 0 009 555 A1

ten Alkylrest mit bis zu 8 Kohlenstoffatomen,

$R^9$ Wasserstoff, einen unverzweigten oder verzweigten Alkylrest mit bis zu 8 Kohlenstoffatomen einen Alkoxyalkylrest mit bis zu 6 Kohlenstoffatomen oder einen Dialkoxyalkylrest mit bis zu 8 Kohlenstoffatomen bedeuten und

$R^8$ und $R^9$ zusammen eine Methylenkette mit 4 oder 5 Kohlenstoffatomen bilden können,

als antagonistischem Mittel.

Das Anteilsverhältnis Äthyl-N-äthyl-N-bicyclo [2.2.1]hept-2-yl-thiolcarbamat : antagonistischem Mittel beträgt dabei bei gemeinsamer oder getrennter Ausbringung 1 : 1 bis 1 : 0,01 Gewichtsteile.

BASF Aktiengesellschaft O. Z. 0050/033330

BEZEICHNUNG GEÄNDERT
siehe Titelseite

Herbizide Mittel auf der Basis eines Thiolcarbamats, die Halogenacylamide als Antagonisten enthalten

Die vorliegende Erfindung betrifft herbizide Mittel, die ein Thiolcarbamat als herbiziden Wirkstoff und Halogenacylamide als antagonistische Mittel enthalten, sowie Verfahren zur selektiven Bekämpfung unerwünschten Pflanzenwuchses mit diesen herbiziden Mitteln.

Die DE-AS 19 53 262 lehrt, daß Äthyl-N-äthyl-N-bicyclo-[2.2.1]hept-2-yl-thiolcarbamat als selektives Herbizid, vorwiegend in Zuckerrüben, wirksam ist. Der Wirkstoff hat eine sehr gute herbizide Wirkung gegen zahlreiche ein- und zweikeimblättrige unerwünschte Pflanzen. Er eignet sich zur Unkrautbekämpfung in Mais, wenn die Selektivitätsmarge etwas vergrößert wird. Man versteht darunter die Spanne in der Aufwandmenge, welche einerseits für eine gute herbizide Wirkung notwendig ist und andererseits von den Kulturpflanzen gerade noch schadlos toleriert wird.

Weiterhin ist aus der DE-AS 22 18 097 bekannt, daß Halogenacetamide bei Anwendung in Kombination mit herbiziden Thiolcarbamaten die Verträglichkeit dieser Wirkstoffe für bestimmte Kulturen verbessern.

H/ML

Es wurde nun gefunden, daß herbizide Mittel, die Äthyl-N-äthyl-N-bicyclo[2.2.1]hept-2-yl-thiolcarbamat der Formel

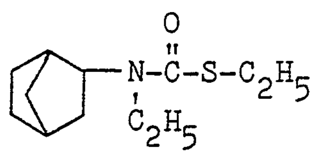

als herbiziden Wirkstoff und mindestens ein Halogenacylamid der Formel I

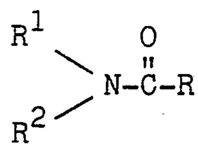

I,

in der

R einen unverzweigten oder verzweigten Halogenalkylrest mit bis zu 3 Kohlenstoffatomen und

$R^1$ und $R^2$ gleich oder verschieden sind und einen unverzweigten oder verzweigten Alkylrest mit bis zu 6 Kohlenstoffatomen, der durch eine Alkoxygruppe mit bis zu 4 Kohlenstoffatomen oder eine Cyanogruppe substituiert sein kann, einen Cycloalkylrest mit 3 bis 6 Kohlenstoffatomen oder einen unverzweigten oder verzweigten Alkenyl- oder Alkinylrest mit bis zu 4 Kohlenstoffatomen bedeuten oder

$R^1$ und $R^2$ zusammen mit dem Stickstoffatom, dessen Substituenten sie sind, einen 4- bis 9-gliedrigen, gesättigten mono- oder bicyclischen Ring, der durch unverzweigte oder verzweigte Alkylreste mit bis zu 4 Kohlenstoffatomen einfach oder mehrfach substituiert sein kann, oder einen Tetrahydro-1,3-oxazinring der Formel

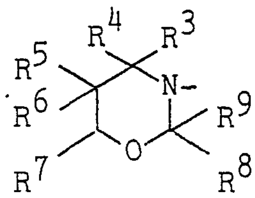

bilden,
in der

$R^3$, $R^4$, $R^5$, $R^6$ und $R^7$ gleich oder verschieden sind und Wasserstoff oder einen unverzweigten oder verzweigten Alkylrest mit bis zu 3 Kohlenstoffatomen,

$R^8$ Wasserstoff oder einen unverzweigten oder verzweigten Alkylrest mit bis zu 8 Kohlenstoffatomen,

$R^9$ Wasserstoff, einen unverzweigten oder verzweigten Alkylrest mit bis zu 8 Kohlenstoffatomen einen Alkoxyalkylrest mit bis zu 6 Kohlenstoffatomen oder einen Dialkoxyalkylrest mit bis zu 8 Kohlenstoffatomen bedeuten und

$R^8$ und $R^9$ zusammen eine Methylenkette mit 4 oder 5 Kohlenstoffatomen bilden können,

als antagonistisches Mittel enthalten, eine wesentlich bessere Verträglichkeit für Kulturpflanzen, wie Mais, haben als herbizide Mittel, die Äthyl-N-äthyl-N-bicyclo[2.2.1]hept-2-yl-thiolcarbamat ohne Zusatz eines antagonistischen Mittels enthalten. Dabei bleibt die gute herbizide Wirkung des Thiolcarbamats erhalten.

Die antagonistisch wirkenden Verbindungen der Formel I haben selbst keinen oder einen kaum sichtbaren Einfluß auf Keimung und Wachstum sowohl von Kulturpflanzen als auch von unerwünschten Pflanzen, selbst bei Aufwandmengen, welche beträchtlich über denjenigen liegen, welche zur Erzielung eines antagonistischen Effektes benötigt werden. Sie haben jedoch die Fähigkeit, die Phytotoxizität des Thiolcarbamats gegenüber Kulturpflanzen, wie Mais, beachtlich zu reduzieren oder völlig zu eliminieren.

Als antagonistisch wirkende Halogenacylamide kommen Verbindungen der Formel I in Betracht, bei denen R Halogenalkyl, insbesondere Chloralkyl, beispielsweise Chlormethyl, Dichlormethyl, Trichlormethyl, 2-Chloräthyl, vorzugsweise Dichlormethyl, bedeutet.

Die Substituenten $R^1$ und $R^2$ können gleich oder verschieden sein und einen unverzweigten oder verzweigten Alkylrest mit bis zu 6 Kohlenstoffatomen, beispielsweise Methyl, Äthyl, n-Propyl, i-Propyl, n-Butyl, i-Butyl, sec-Butyl, tert-Butyl, n-Hexyl, 1,4-Dimethyl-n-butyl, einen unverzweigten oder verzweigten Alkenyl- oder Alkinylrest mit bis zu 4 Kohlenstoffatomen, beispielsweise Allyl, Propargyl, 1-Methyl-butin-2-yl, oder einen Cycloalkylrest mit 3 bis 6 Kohlenstoffatomen, beispielsweise Cyclopropyl, Cyclohexyl bedeuten. Die Alkylreste können gegebenenfalls durch eine Alkoxygruppe mit bis zu 4 Kohlenstoffatomen, beispielsweise durch Methoxy, Äthoxy, oder durch eine Cyanogruppe substituiert sein. Die Alkoxygruppe steht vorzugsweise in Endstellung der Kohlenstoffreste.

$R^1$ und $R^2$ können außerdem zusammen mit dem Stickstoffatom, dessen Substituenten sie sind, einen 4- bis 9-gliedrigen gesättigten mono- oder bicyclischen Ring bilden. Dieser Heterocyclus kann gegebenenfalls durch unverzweigte oder verzweigte Alkylreste mit bis zu 4 Kohlenstoffatomen einfach oder mehrfach substituiert sein. Beispiele für diese Ringe sind Piperidinyl, Alkylpiperidinyl, beispielsweise 3,5-Diäthylpiperidinyl, 2,5-Dimethyl-pyrrolidinyl, Azetidinyl, Alkyl-azetidinyl, beispielsweise 2.2.4-Trimethylazetidinyl, Hexahydroazepinyl, Alkyl-hexahydroazipinyl, beispielsweise 3.5.5/3.3.5-Trimethyl-hexahydro-azepinyl, 2,3-Dimethyl-hexahydroazepinyl, Aza-bicyclo[3.2.2]nonyl, Alkyl-aza-bicyclo-octyl, beispielsweise Trimethyl-aza-bicyclo[3.2.1]-octyl, Aza-bicyclo[3.2.0]heptyl.

Darüber hinaus können $R^1$ und $R^2$ zusammen mit dem Stickstoffatom, dessen Substituenten sie sind, einen Tetrahydro-1,3-oxazinring der Formel

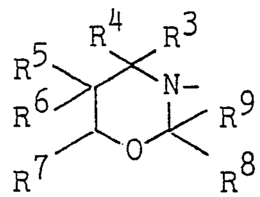

bilden. Die Substituenten $R^3$ bis $R^7$ können gleich oder verschieden sein und Wasserstoff oder Alkyl mit bis zu 3 Kohlenstoffatomen, insbesondere Wasserstoff oder Methyl, bedeuten. $R^8$ und $R^9$ bedeuten Wasserstoff oder Alkylreste mit bis zu 8 Kohlenstoffatomen, wie Methyl-, Äthyl, n-Propyl, i-Propyl, Butyl-, Hexyl-, Heptyl- oder Octylreste.

$R^9$ kann auch für Alkoxyalkylreste mit bis zu 6 Kohlenstoffatomen oder für Dialkoxyalkylreste mit bis zu 8 Kohlenstoffatomen stehen, beispielsweise für Methoxymethyl, Dimethoxymethyl. $R^8$ und $R^9$ können zusammen eine Methylenkette mit 4 oder 5 Kohlenstoffatomen bilden. R bedeutet Halogenalkyl mit bis zu 3 Kohlenstoffatomen, vorzugsweise Chloralkyl, insbesondere Chlormethyl oder Dichlormethyl. Diese N-Halogenacylamid-tetrahydro-1,3-oxazine sind neu.

Als Antagonisten bevorzugte Halogenacylamide der Formel I sind N-Isopropyl-N-propargyl-dichloracetamid, N-Dichloracetyl-4,4-dimethyl-tetrahydro-1,3-oxazin, N-Dichloracetyl-4,4,6-trimethyl-tetrahydro-1,3-oxazin und N,N-Diallyl-dichloracetamid.

Die Halogenacylamide der Formel I können durch Umsetzung der Amine der Formel

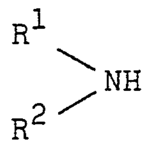

in der $R^1$ und $R^2$ die oben genannten Bedeutungen haben, mit Halogenacylchlorid . erhalten werden. Die Umsetzung wird in an sich bekannter Weise in Gegenwart eines Chlorwasserstoff-bindenden Mittels in einem inerten Lösungs- oder Verdünnungsmittel durchgeführt.

Die folgenden Beispiele erläutern die Herstellung der Halogenacylamide:

<u>Beispiel 1</u>

118 g Isopropyl-propargyl-amin und 123 g Triäthylamin werden in 670 ml Toluol gelöst. Zu dieser Lösung tropft man unter Kühlung bei Raumtemperatur 180 g Dichloracetylchlorid, gelöst in 450 ml Toluol, zu. Man läßt eine Stunde lang nachreagieren, saugt ab und wäscht das Filtrat mit Wasser. Der nach dem Abziehen des Lösungsmittels verbleibende Rückstand wird mit Petroläther gewaschen. Man erhält 214 g N-Isopropyl-N-propargyl-dichloracetamid, entsprechend einer Ausbeute von 84 % d.Th.; Kp.:80-82°C/0,013 mbar; Schmp.: 58 bis 59°C.

<u>Beispiel 2</u>

Zu 23,0 Gewichtsteilen 4,4-Dimethyl-tetrahydro-1,3-oxazin und 20,7 Gewichtsteilen Triäthylamin in 100 Volumenteilen Toluol werden bei -10°C 23,2 Gewichtsteile Dichloracetylchlorid in 100 Volumenteilen Toluol unter Rühren zugetropft. Nach 2-stündigem Nachrühren bei Raumtemperatur werden 150 Volumenteile Methylenchlorid und so viel Wasser zugesetzt, daß zwei klare Phasen entstehen. Die organische

Phase wird abgetrennt und zweimal mit je 50 Volumenteilen Wasser gewaschen. Nach dem Trocknen und Verdampfen der Lösungsmittel unter vermindertem Druck isoliert man 41 Gewichtsteile N-Dichloracetyl-4,4-dimethyl-tetrahydro-1,3-oxazin vom Fp. 105 bis 106°C, das nach Umkristallisieren aus Methanol bei 106 bis 107°C schmilzt.

$C_8H_{13}N_2O_2Cl_2$ MG 226

| | | | |
|---|---|---|---|
| ber.: | C 42,5 | H 5,8 | N 6,19 |
| gef.: | C 42,6 | H 5,8 | N 6,2 |

In analoger Weise können folgende Verbindungen hergestellt werden:

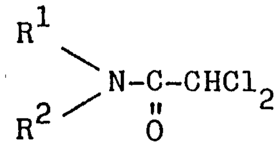

| $R^1$ | $R^2$ | $Kp/Fp/n_D$ | |
|---|---|---|---|
| $CH_2=CH-CH_2-$ | $CH_2=CH-CH_2-$ | $n_D^{30}$: | 1,4990 |
| $HC\equiv C-CH_2-$ | $n-C_3H_7$ | $n_D^{26}$: | 1,4978 |
| $HC\equiv C-CH_2-$ | $(CH_3)_2CH-CH_2-CH(CH_3)-$ | $n_D^{25}$: | 1,4889 |
| $HC\equiv C-CH_2-$ | $CH_3$ | $n_D^{25}$: | 1,5090 |
| $i-C_3H_7$ | $C_2H_5$ | $n_D^{25}$: | 1,4849 |
| $i-C_3H_7$ | $i-C_3H_7$ | Fp: | 62- 65°C |
| $n-C_4H_9$ | $C_2H_5$ | $n_D^{25}$: | 1,4802 |
| sec.-$C_4H_9$ | $CH_3$ | $n_D^{25}$: | 1,4820 |
| $i-C_4H_9$ | $CH_3$ | $n_D^{25}$: | 1,4820 |
| sec.-$C_4H_9$ | $HC\equiv C-CH(CH_3)-$ | $n_D^{25}$: | 1,4923 |
| $n-C_4H_9$ | $CH_3$ | $n_D^{25}$: | 1,4835 |
| $HC\equiv C-CH_2-$ | sec.-$C_4H_9$ | $n_D^{25}$: | 1,4960 |

| $R^1$ | $R^2$ | Kp/Fp/$n_D$ | |
|---|---|---|---|
| | $-(CH_2)_3-$ | Fp: | 37- 39°C |
| | $-(CH_2)_4-$ | $n_D^{25}$: | 1,5190 |
| | $-(CH_2)_5-$ | Fp: | 41- 42°C |
| | 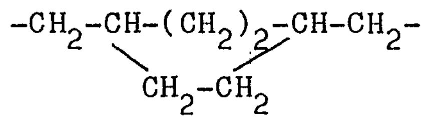 | Fp: | 101-102°C |
| | $-CH(CH_3)-CH_2-C(CH_3)_2-$ | Fp: | 48- 49°C |
| | 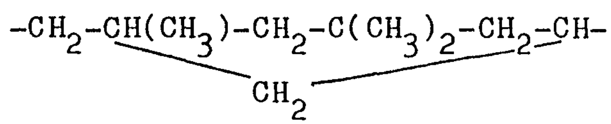 | Fp: | 82- 84°C |
| | $-CH(CH_3)-(CH_2)_2-CH(CH_3)-$ | Fp: | 61- 65°C |
| | $-CH_2-CH(C_2H_5)-CH_2-CH(C_2H_5)-CH_2-$ | $n_D^{25}$: | 1,5003 |
| $H_3CO-CH_2-CH_2$ | $H_3CO-CH_2-CH_2-$ | Kp: | 132°C/0,4 mbar |
| $H_3CO-CH_2-CH(CH_3)-$ | $CH_3$ | Kp: | 105°C/0,53 mbar |
| $H_3CO-CH_2-CH(CH_3)-$ | i-$C_3H_7$ | Kp: | 104°C/0,67 mbar |
| 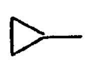 | $-CH_2-C{\equiv}CH$ | Fp: | 73- 75°C |
| tert.-$C_4H_9$ | $-CH_2-C{\equiv}CH$ | $n_D^{25}$: | 1,4950 |

| $R^1$ | $R^2$ | $Kp/Fp/n_D$ | |
|---|---|---|---|
| $i-C_3H_7$ | 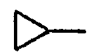 | $n_D^{25}$: | 1,4936 |
| $C_6H_{11}$ | 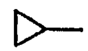 | Fp: | 104-106°C |
| $C_6H_{11}$ | $-CH_2-CN$ | Fp: | 98-103°C |
| $C_2H_5-CH(CH_3)-$ | $-CH_2-CN$ | $n_D^{25}$: | 1,5028 |
| tert.-$C_4H_9$ | $-CH_2-CN$ | Fp: | 117-119°C |
| | 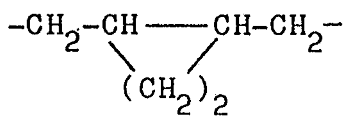 | Kp: | 110-112°C/0,013 mbar |

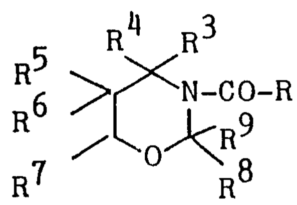

| R | $R^3$ | $R^4$ | $R^5$ | $R^6$ | $R^7$ | $R^8$ | $R^9$ | $Fp^oC/Kp^oC/n_D^{25}$ |
|---|---|---|---|---|---|---|---|---|
| $CH_2Cl$ | $CH_3$ | $CH_3$ | H | H | H | H | H | Kp: $90^oC/0{,}067$ mbar |
| $CHCl_2$ | $CH_3$ | $CH_3$ | H | H | H | H | H | Fp: $108^oC$ |
| $CHCl_2$ | $CH_3$ | $CH_3$ | H | H | H | $CH_3$ | H | Öl |
| $CHCl_2$ | $CH_3$ | $CH_3$ | H | H | $CH_3$ | H | H | Fp: $56^oC$ |
| $CHCl_2$ | $CH_3$ | $CH_3$ | H | H | $CH_3$ | $CH_3$ | H | 1,4918 |
| $CHCl_2$ | $CH_3$ | $CH_3$ | H | H | $CH_3$ | $C_2H_5$ | H | 1,4949 |
| $CHCl_2$ | $CH_3$ | $CH_3$ | H | H | $CH_3$ | $n-C_3H_7$ | H | 1,4915 |
| $CHCl_2$ | $CH_3$ | $CH_3$ | H | H | $CH_3$ | $i-C_3H_7$ | H | 1,4945 |
| $CHCl_2$ | H | H | $CH_3$ | $CH_3$ | H | H | H | Fp: $64^oC$ |
| $CHCl_2$ | H | H | $CH_3$ | $CH_3$ | H | $CH_3$ | H | Fp: $80^oC$ |
| $CHCl_2$ | H | H | $CH_3$ | $CH_3$ | H | $i-C_3H_7$ | H | Fp: $84^oC$ |

| R | $R^3$ | $R^4$ | $R^5$ | $R^6$ | $R^7$ | $R^8$ | $R^9$ | Fp°C/Kp°C/$n_D^{25}$ |
|---|---|---|---|---|---|---|---|---|
| $CHCl_2$ | $CH_3$ | $CH_3$ | H | H | $CH_3$ | $CH(C_2H_5)-nC_4H_9$ | H | 1,4849 |
| $CHCl_2$ | $CH_3$ | $CH_3$ | H | H | $CH_3$ | H | $CH(OCH_3)_2$ | Öl |
| $CHCl_2$ | $CH_3$ | $CH_3$ | H | H | $CH_3$ | $CH_3$ | $CH_3OCH_2$ | Öl |
| $CHCl_2$ | $CH_3$ | $CH_3$ | H | H | $CH_3$ | $-(CH_2)_4-$ | | Öl |
| $CHCl_2$ | $CH_3$ | $CH_3$ | H | H | $CH_3$ | $-(CH_2)_5-$ | | Öl |
| $CCl_3$ | $CH_3$ | $CH_3$ | H | H | H | H | H | Fp: 103°C |
| $CHCl_2$ | $CH_3$ | $CH_3$ | H | H | H | $C_2H_5$ | H | 1,5152 |
| $CHCl_2$ | $CH_3$ | $CH_3$ | H | H | H | $n-C_3H_7$ | H | 1,5010 |

Äthyl-N-äthyl-N-bicyclo[2.2.1]hept-2-yl-thiolcarbamat kann durch Umsetzung von Äthyl-bicyclo[2.2.1]hept-2-yl-amin mit Chlorameisensäureäthylthiolester in Gegenwart eines säurebindenden Mittels erhalten werden.

Herbizider Wirkstoff und antagonistisch wirkende Verbindungen, können gemeinsam oder getrennt vor oder nach der Saat in den Boden eingearbeitet werden. Ebenso können sie gemeinsam oder getrennt vor oder nach der Saat, aber vor dem Auflaufen der Kulturpflanzen auf die Oberfläche des zu behandelnden Feldes ausgebracht werden. Wirkstoff und Antagonist können hierbei als Spritzmittel in suspendierbarer, emulgierbarer oder löslicher Form oder als Granulate getrennt oder gemeinsam formuliert vorliegen. Denkbar ist auch eine Behandlung der Kulturpflanzensamen mit dem Antagonisten vor der Aussaat. Der herbizide Wirkstoff wird dann allein in der üblichen Weise appliziert.

Das Anteilsverhältnis Thiolcarbamat : Halogenacylamid kann, bei gemeinsamer oder getrennter Ausbringung, stark schwanken. Üblicherweise liegen die Anteilsverhältnisse Thiolcarbamat : Halogenacylamid zwischen 1 : 1 bis 1 : 0,01 Gewichtsteilen.

Die neuen herbiziden Mittel können neben Thiolcarbamat und Antagonist weitere herbizide oder wachstumsregulierende Wirkstoffe anderer chemischer Struktur, beispielsweise 2-Chlor-4-äthylamino-6-isopropylamino-1,3,5-triazin, enthalten, ohne daß der antagonistische Effekt der Halogenacetamide beeinflußt wird.

Die erfindungsgemäßen Mittel bzw. bei getrennter Ausbringung der herbizide Wirkstoff oder das Antidot werden beispielsweise in Form von direkt versprühbaren Lösungen, Pulvern, Suspensionen, auch hochprozentige wäßrige, ölige

oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und evtl. Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate,

Alkylsulfonate, Alkali- und Erdalkalisalze der Dibutylnaphthalinsulfonsäure, Lauryläthersulfat, Fettalkoholsulfate, fettsäure Alkali- und Erdalkalisalze, Salze sulfatierter Hexadecanole, Heptadecanole, Octadecanole, Salze von sulfatierten Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol-, Octylphenol, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gew.% an herbizidem Wirkstoff und Antidot, vorzugsweise zwischen 0,5 und 90 Gew.%. Die Aufwandmengen an herbizidem Wirkstoff betragen 0,2 bis 5 kg/ha. Diese Menge an herbizidem Wirkstoff wird, gemeinsam oder getrennt, mit einer solchen Menge an Antidot ausgebracht, daß das Anteilsverhältnis herbizider Wirkstoff : antagonistischer Verbindung 1 : 1 bis 1 : 0,01 Gewichtsteile beträgt.

Beispiele für Formulierungen sind:

I. 40 Gewichtsteile der Mischung aus sechs Gewichtsteilen Äthyl-N-äthyl-N-bicyclo[2.2.1]hept-2-yl-thiolcarbamat und einem Gewichtsteil N-Dichloracetyl-2,2-dimethyl-tetrahydro-1,3-oxazin werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff und Antidot enthält.

II. 3 Gewichtsteile der Mischung aus drei Gewichtsteilen Äthyl-N-äthyl-N-bicyclo[2.2.1]hept-2-yl-thiolcarbamat und einem Gewichtsteile N-Dichloracetyl-4,4,6-trimethyl-tetrahydro-1,3-oxazin werden mit 97 Gewichtsteilen feinteiligem Kaolin innig vermischt. Man erhält auf diese Weise ein Stäubmittel, das 3 Gew.% ~~des~~ Wirkstoff und Antidot enthält.

III. 30 Gewichtsteile der Mischung aus drei Gewichtsteilen Äthyl-N-äthyl-N-bicyclo[2.2.1]hept-2-yl-thiolcarbamat und einem Gewichtsteil N,N-Diallyl-dichloracetamid werden mit einer Mischung aus 92 Gewichtsteilen pulverförmigem Kieselsäuregel und 8 Gewichtsteilen

Paraffinöl, das auf die Oberfläche dieses Kieselsäuregels gesprüht wurde, innig vermischt.

IV. 20 Teile der Mischung aus sechs Gewichtsteilen Äthyl-N-äthyl-N-bicyclo[2.2.1]hept-2-yl-thiolcarbamat und einem Gewichtsteil N-Isopropyl-N-propargyl-dichloracetamid werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Der Einfluß der erfindungsgemäßen herbiziden Mittel auf das Wachstum von Mais und unerwünschten Pflanzen wird durch die folgenden biologischen Beispiele belegt:

Plastikpikierkisten von 51 cm Länge, 32 cm Breite und 6 cm Höhe wurden mit lehmigem Sand von pH 6 und etwa 1,5 % Humus gefüllt. In dieses Substrat wurde Mais (Zea mays) als Kulturpflanze in Reihen flach eingesät. Außerdem wurden Hühnerhirse (Echinochloa crus galli) und Ackerfuchsschwanz (Alopecurus myosuroides) als unerwünschte Pflanzen breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simuliert.

Der Wirkstoff und die antagonistisch wirkenden Verbindungen wurden sowohl einzeln als auch in den beschriebenen Mischungen ausgebracht. Sie wurden, emulgiert oder suspendiert in Wasser als Trägermedium, mittels fein verteilender Düsen unmittelbar nach der Saat und vor Auflaufen der Testpflanzen auf die Erdoberfläche gespritzt. Nach Einsaat

und Behandlung wurden die Kisten beregnet und bis nach dem Auflaufen der Pflanzen mit durchsichtigen Plastikhauben abgedeckt. Durch diese Maßnahme wurde ein gleichmäßiges Keimen und Anwachsen der Pflanzen garantiert. Die Aufstellung erfolgte in einem Temperaturbereich von durchschnittlich 18 bis 30°C.

Die so angelegten Gewächshausversuche wurden beobachtet, bis der Mais 3 bis 5 Blätter entwickelt hatte. Nach diesem Stadium waren bei den herbiziden Mitteln keine Schädigungen mehr zu erwarten.

Die erfindungsgemäßen Mittel bzw. des Wirkstoffs oder der Antagonisten wurde nach einer Skala von 0 bis 100 bewertet. Hierbei bedeutet 0 normaler Aufgang und normale Entwicklung der Pflanzen, bezogen auf die unbehandelte Kontrolle. 100 entspricht einem völligen Ausbleiben der Keimung bzw. Absterben der Pflanzen. Es mußte dabei berücksichtigt werden, daß beispielsweise bei Mais auch unter völlig normalen Verhältnissen und ohne jede chemische Behandlung vereinzelt verkrüppelte oder gehemmte Pflanzen vorkommen.

In der folgenden Tabelle steht
A für Äthyl-N-äthyl-N-bicyclo[2.2.1]hept-2-yl-thiolcarbamat,
B für N-Isopropyl-N-propargyl-dichloracetamid,
C für N-Dichloracetyl-4,4-dimethyl-tetrahydro-1,3-oxazin.

Die Versuchsergebnisse zeigen, daß die Verträglichkeit von Äthyl-N-äthyl-N-bicyclo[2.2.1]hept-2-yl-thiolcarbamat für die Kulturpflanze Mais mittels antagonistisch wirkenden Verbindungen der Formel I ganz erheblich gesteigert wird.

Tabelle - Verbesserung der Maisverträglichkeit von Äthyl-N-äthyl-N-bicyclo[2.2.1]hept-2-yl-thiolcarbamat durch antagonistisch wirkende Verbindungen bei Vorauflaufanwendung im Gewächshaus.

| Herbizider Wirkstoff | | Antagonistische Verbindung | Aufwandmenge kg/ha a.S. | Testpflanzen und % Schädigung | | |
|---|---|---|---|---|---|---|
| | | | | Zea mays | Alopecurus myosuroides | Echinochloa crus galli |
| A | | - | 3,0 | 20 | 98 | 96 |
| A | | - | 6,0 | 25 | 98 | 98 |
| | | C | 4,0 | 5 | - | 0 |
| | | B | 2,0 | 0 | 0 | 0 |
| A | + | C | 6,0+1,0 | 5 | - | 95 |
| | | | 3,0+0,5 | 5 | - | 95 |
| A | + | B | 6,0+3,0 | 5 | - | 98 |
| | | | 6,0+2,0 | 2,5 | 98 | 98 |
| | | | 6,0+1,0 | 5 | 100 | 97 |
| | | | 3,0+0,5 | 0 | 99 | 97 |
| | | | 3,0+0,25 | 2,5 | 98 | 98 |

## Patentansprüche

1. Herbizides Mittel, gekennzeichnet durch einen Gehalt an Äthyl-N-äthyl-N-bicyclo[2.2.1]hept-2-yl-thiolcarbamat der Formel

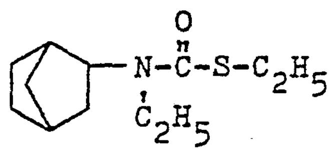

als herbizidem Wirkstoff und mindestens einem Halogenacylamid der Formel I

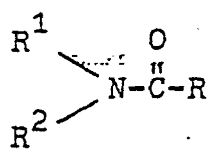

I,

in der

R einen unverzweigten oder verzweigten Halogenalkylrest mit bis zu 3 Kohlenstoffatomen und

$R^1$ und $R^2$ gleich oder verschieden sind und einen unverzweigten oder verzweigten Alkylrest mit bis zu 6 Kohlenstoffatomen, der durch eine Alkoxygruppe mit bis zu 4 Kohlenstoffatomen oder eine Cyanogruppe substituiert sein kann, einen Cycloalkylrest mit 3 bis 6 Kohlenstoffatomen oder einen unverzweigten oder verzweigten Alkenyl- oder Alkinylrest mit bis zu 4 Kohlenstoffatomen bedeuten oder

$R^1$ und $R^2$ zusammen mit dem Stickstoffatom, dessen Substituenten sie sind, einen 4- bis 9-gliedrigen, gesättigten mono- oder bicyclischen Ring, der durch unverzweigte oder verzweigte Alkylreste mit bis zu

4 Kohlenstoffatomen einfach oder mehrfach substituiert sein kann, oder einen Tetrahydro-1,3-oxazinring der Formel

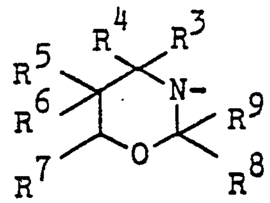

bilden,
in der
$R^3$, $R^4$, $R^5$, $R^6$ und $R^7$ gleich oder verschieden sind und Wasserstoff oder einen unverzweigten oder verzweigten Alkylrest mit bis zu 3 Kohlenstoffatomen,
$R^8$ Wasserstoff oder einen unverzweigten oder verzweigten Alkylrest mit bis zu 8 Kohlenstoffatomen,
$R^9$ Wasserstoff, einen unverzweigten oder verzweigten Alkylrest mit bis zu 8 Kohlenstoffatomen einen Alkoxyalkylrest mit bis zu 6 Kohlenstoffatomen oder einen Dialkoxyalkylrest mit bis zu 8 Kohlenstoffatomen bedeuten und
$R^8$ und $R^9$ zusammen eine Methylenkette mit 4 oder 5 Kohlenstoffatomen bilden können,
als antagonistischem Mittel.

2. Herbizides Mittel nach Anspruch 1, dadurch gekennzeichnet, daß es als antagonistisches Mittel N-Isopropyl-N--propargyl-dichloracetamid enthält.

3. Herbizides Mittel nach Anspruch 1, dadurch gekennzeichnet, daß es als antagonistisches Mittel N-Dichloracetyl-4,4-dimethyl-tetrahydro-1,3-oxazin enthält.

4. Herbizides Mittel nach Anspruch 1, dadurch gekennzeichnet, daß das Anteilsverhältnis Äthyl-N-äthyl-N-bicyclo-[2.2.1]hept-2-yl-thiolcarbamat : antagonistischem Mittel 1 : 1 bis 1 : 0,01 Gewichtsteile beträgt.

5. Verfahren zur selektiven Bekämpfung von unerwünschtem Pflanzenwuchs, dadurch gekennzeichnet, daß man herbizide Mittel nach Anspruch 1 vor, bei oder nach der Aussaat der Kulturpflanzen, vor oder während des Auflaufens der Kulturpflanzen ausbringt.

6. Verfahren zur selektiven Bekämpfung von unerwünschtem Pflanzenwuchs, dadurch gekennzeichnet, daß man Äthyl-N-äthyl-N-bicyclo[2.2.1]hept-2-yl-thiolcarbamat und das antagonistische Mittel vor, bei oder nach der Aussaat der Kulturpflanzen, vor oder während des Auflaufens der Kulturpflanzen gleichzeitig oder nacheinander in beliebiger Reihenfolge, mit oder ohne Einarbeitung, ausbringt.

7. Verfahren nach Anspruch 6, dadurch gekennzeichnet, daß das Anteilsverhältnis Äthyl-N-äthyl-N-bicyclo-2.2.1 hept-2-yl-thiolcarbamat : antagonistischem Mittel bei getrennter Ausbringung 1 : 1 bis 1 : 0,01 Gewichtsteile beträgt.

8. Verfahren nach Anspruch 5 und 6, dadurch gekennzeichnet, daß die Kulturpflanze Mais ist.

Europäisches Patentamt

# EUROPÄISCHER RECHERCHENBERICHT

0009555
Nummer der Anmeldung

EP 79 10 2572

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | US - A - 4 021 224 (F.M. PALLOS et al.)<br>* Spalte 1, Zeile 34 bis Spalte 2, Zeile 40; Spalte 87, Anspruch 8 * | 1-8 | A 01 N 47/18<br>25/32<br>C 07 C 103/34<br>121/43<br>C 07 D 205/04<br>207/06<br>209/02<br>209/52<br>211/16<br>265/04<br>295/18 |
| D | & DE - A - 2 218 097<br>& FR - A - 2 133 793<br>-- | | |
| D | DE - A - 1 953 262 (BASF)<br>* Seite 5, Beispiel 2; Anspruch 2 *<br>-- | 1-8 | RECHERCHIERTE SACHGEBIETE (Int. Cl. 3) |
| | FR - A - 2 153 002 (GULF)<br>* Seite 1, Zeilen 20-35; Ansprüche *<br>& DE - A - 2 245 471<br>-- | 1-8 | A 01 N 25/32<br>47/18 |
| A | FR - A - 2 310 348 (PRODUITS CHIMIQUES UGINE KUHLMANN)<br>---- | | |

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

| | |
|---|---|
| ℘ | Der vorliegende Recherchenbericht wurde für alle Patentansprüche erstellt. |

| Recherchenort | Abschlußdatum der Recherche | Prüfer |
|---|---|---|
| Den Haag | 23-10-1979 | DECORTE |